(19) Europäisches Patentamt
European Patent Office
Office européen des brevets

(11) Veröffentlichungsnummer: **0 132 737**
**B1**

(12) # EUROPÄISCHE PATENTSCHRIFT

(45) Veröffentlichungstag der Patentschrift: **07.01.88**

(51) Int. Cl.⁴: **C 07 C 67/34**, C 07 C 69/025

(21) Anmeldenummer: **84108310.8**

(22) Anmeldetag: **14.07.84**

(54) **Verfahren zur Isomerisierung von Diacyloxy-butenen.**

(30) Priorität: **23.07.83 DE 3326668**

(43) Veröffentlichungstag der Anmeldung:
**13.02.85 Patentblatt 85/7**

(45) Bekanntmachung des Hinweises auf die Patenterteilung:
**07.01.88 Patentblatt 88/1**

(84) Benannte Vertragsstaaten:
**BE CH DE FR GB IT LI NL**

(56) Entgegenhaltungen:
**DE - A - 3 022 288**

**CHEMICAL ABSTRACTS, Band 84, Nr. 5, 2. Februar 1976, Columbus, Ohio, USA; Seite 410, Spalte 1, Zusammenfassung Nr. 30473h; & JP-A-75-126 611 (MITSUBISHI CHEMICAL INDUST RIES) (04-10-1985 (Kat. A,D)**
**PATENT ABSTRACTS OF JAPAN, Band 5, Nr. 45, 25. März 1981, Seite (C-48)(717); & JP-A-55-167 253 (NI PPON GOSEI GOMU) 26-12-1980**
**CHEMICAL ABSTRACTS, Band 102, Nr. 6, 11. Februar 1985, Columbus, Ohio, USA; B.S. RAO et al. "Isomerization of alkylaromatics over ferrosilicate zeolites", Seite 105, Spalte 2, Zusammenfassung Nr. 47705y; & Actas Simp. Iberoam. Catal., Band 9, 1984, Nr.**

(73) Patentinhaber: **BASF Aktiengesellschaft, Carl-Bosch-Strasse 38, D-6700 Ludwigshafen (DE)**

(72) Erfinder: **Hoelderich, Wolfgang, Dr., Mannheimer Strasse 18 c, D-6710 Frankenthal (DE)**
Erfinder: **Fischer, Rolf, Dr., Bergstrasse 98, D-6900 Heidelberg (DE)**
Erfinder: **Hertel, Otto, Dr., Luitpoldstrasse 33, D-6700 Ludwigshafen (DE)**
Erfinder: **Mross, Wolf Dieter, Dr., Anselm-Feuerbach-Strasse 21, D-6710 Frankenthal (DE)**
Erfinder: **Weitz, Hans-Martin, Dr., Auf dem Koeppel 40, D-6702 Bad Duerkheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein Verfahren zur wechselseitigen Umwandlung von 1,4-Diacyloxy-2-butenen (I) und 3,4-Diacyloxy-1-butenen (II) in der Gasphase oder in der Flüssigphase in Gegenwart von Zeolithen.

Diacyloxybutene der Formel I und II stellen wertvolle Zwischenprodukte dar. So lässt sich z.B. 1,4-Diacetoxy-2-buten nach Hydrierung und Verseifung in 1,4-Butandiol und Tetrahydrofuran überführen. 3,4-Diacyloxy-1-butene können nach Hydroformylierung und Abspaltung von einem Mol Essigsäure zu trans-2-Methyl-4-acetoxy-2-butenal, einem Synthesebaustein für Terpene, wie z.B. Vitamin A-Acetat, umgesetzt werden.

Gemische von Diacyloxybutenen der Formel I und II lassen sich durch Umsetzung von Butadien oder substituierten 1,3-Dienen mit Carbonsäuren und Sauerstoff in Gegenwart von Palladium oder Platin enthaltenden Katalysatoren herstellen.

Es ist bekannt, 3,4-Diacyloxy-1-butene und 1,4-Diacyloxy-2-butene durch Erhitzen in der Flüssigphase in Gegenwart von homogen gelösten Metallverbindungen ineinander umzuwandeln. Hierfür wurden z.B. Selendioxid in Gegenwart von Lithiumacetat, Essigsäure und Acetanhydrid (U.S. Patent 4 182 901) verwendet. Es ist weiterhin möglich, Palladium- oder Platinverbindungen wie Platin- oder Palladiumhalogenide in Gegenwart von Sauerstoff (DT-OS 24 54 768, 21 34 115) oder Sauerstoff und Chlor (DT-OS 27 36 695) einzusetzen.

Für die genannte Isomerisierung wurden auch schon heterogene Katalysatoren in der Flüssigphase, wie z.B. Kationenaustauscher wie Amberlite 200 C (DT-OS 30 22 288), verwendet.

Als heterogene Katalysatoren in der Gasphase wurden z.B. γ-Aluminiumoxid und/oder Gemische von $SiO_2$ und $AP_2O_3$ (Jap Anm. 50126 611) eingesetzt.

Auch ist es bereits bekannt, dass Zeolithe zur Gerüstisomerisierung von Paraffinen, Olefinen und alkylsubstituierten Aromaten eingesetzt werden. So wird im EP 812 die Xylolisomerisierung an ZSM5-Zeolithen bevorzugt zur Herstellung von p-Xylol aus einem $C_8$-Aromatengemisch beschrieben.

Nachteilig an den bekannten Verfahren zur gegenseitigen Umwandlung von 3,4-Diacyloxy-1-butenen und 1,4-Diacyloxy-2-butenen sind im Falle der in Flüssigphase verwendeten homogenen Katalysatoren deren Abtrennung nach der Umsetzung, deren Rückführung in die Reaktionszone sowie gegebenenfalls deren Regenerierung. Bei den heterogenen, in der Gasphase eingesetzten Katalysatoren sind Selektivität (z.B. 68% bei γ-$Al_2O_3$ bei 40%igem Umsatz) und Standzeit (Pd auf Aktivkohle in Gegenwart von Halogenwasserstoffen) problematisch.

Es wurde nun gefunden, dass sich die wechselseitige Umwandlung der 1,4-Diacyloxy-2-butene der Formeln

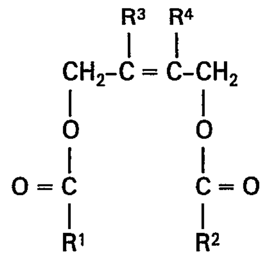

I,

und 3,4-Diacyloxy-1-butene

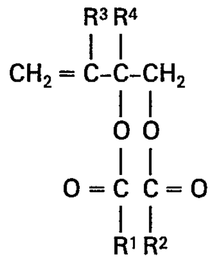

II,

in denen $R^1$ und $R^2$ gleich oder verschieden sein können und Wasserstoff, Alkylreste mit 1 bis 3 Kohlenstoffatomen, $R^3$ und $R^4$ gleich oder verschieden sein können und Wasserstoff oder Methylgruppen bedeuten, dadurch besonders günstig durchführen lässt, dass man in der Gasphase und in der Flüssigphase in Gegenwart von Zeolithen als Katalysatoren arbeitet.

Die wechselseitige Umwandlung der Diacyloxybutene I und II lässt sich für den Fall von cis- und trans-1,4-Diacetoxy-2-buten und 3,4-Diacyloxy-1-buten durch die folgenden Formeln darstellen:

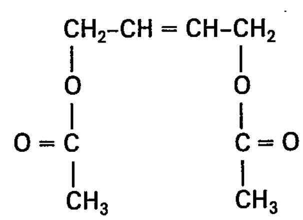

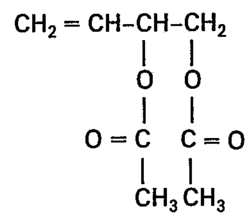

Als Diacyloxybutene I und II, die als reine Verbindungen oder auch als Gemische eingesetzt werden können, sind z.B. cis- und trans-1,4-Diacetoxy-2-buten, 3,4-Diacetoxy-1-buten, 1,4-Diformyloxy-2-buten, 3,4-Diformyloxy-1-buten, 1,4-Diacetoxy-2-methyl-2-buten, 1,4-Dipropionyloxy-2-buten, 3,4-Dipropionyloxy-1-buten, 1,4-Diacetoxy-2,3-dimethyl-2-buten, 3,4-Diacetoxy-3-methyl-1-buten, 3,4-Diacetoxy-2-methyl-1-buten, 3,4-Diacetoxy-2,3-dimethyl-1-buten zu nennen. Besonders bevorzugt hierbei sind cis- und trans-1,4-Diacetoxy-2-buten und 3,4-Diacetoxy-1-buten.

Als Katalysatoren für die Isomerisierung von Diacyloxybutenen werden Zeolithe eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der

Si- und Al-Atome zu Sauerstoff beträgt 1:2. Die Elektrovalenz der Aluminium enthaltenden Tetraeder ist durch Einschluss von Kationen in den Kristall z.B. eines Alkali- oder Wasserstoffions ausgeglichen. Ein Kationenaustausch ist möglich. Die Räume zwischen den Tetraedern sind vor der Dehydratation durch Trocknen bzw. Calcinieren von Wassermolekeln besetzt.

Auch gibt es kristalline Verbindungen mit Zeolithstruktur, bei denen dreiwertige Elemente wie B, Ga, Fe, Cr anstelle des Aluminiums oder vierwertige Elemente wie Ge anstelle des Siliciums in das Zeolithgitter eingebaut sind.

Vorzugsweise werden als Katalysatoren für die Isomerisierung von Diacyloxybutenen Zeolithe des Pentasil-Typs eingesetzt.

Diese Zeolithe können unterschiedliche chemische Zusammensetzung aufweisen. Es handelt sich hierbei um Alumino-, Boro-, Eisen-, Gallium-, Chrom-, Arsen- und Bismutsilikatzeolithe oder deren Gemisch sowie um Alumino-, Boro-, Gallium- und Eisengermanatzeolithe oder deren Gemische.

Insbesondere eignen sich die Alumino- und Borosilikatzeolithe für die beanspruchte Isomerisierung. Der Aluminosilikatzeolith wird aus einer Aluminiumverbindung, vorzugsweise $Al(OH)_3$ oder $Al_2(SO_4)_3$, und einer Siliciumkomponente, vorzugsweise hochdisperses Siliciumdioxid in wässriger Aminlösung, insbesondere in 1,6-Hexandiamin- oder 1,3-Propandiamin- oder Triethylentetramin-Lösung mit und ohne Alkali- oder Erdalkalizusatz bei 100 bis 220°C unter autogenem Druck hergestellt. Die erhaltenen Aluminosilikatzeolithe enthalten je nach Wahl der Einsatzstoffmengen ein $SiO_2/Al_2O_3$-Verhältnis von 10 bis 40 000.

Auch lassen sich derartige Aluminosilikatzeolithe in etherischem Medium wie Diethylenglykoldimethylether, in alkoholischem Medium wie Methanol bzw. 1,4-Butandiol oder lediglich in Wasser synthetisieren.

Der Borosilikatzeolith wird bei 90 bis 170°C unter autogenem Druck synthetisiert, indem man eine Borverbindung, z.B. $H_3BO_3$, mit einer Siliciumverbindung, vorzugsweise hochdisperses Siliciumdioxid in wässriger Aminlösung, insbesondere in 1,6-Hexandiamin- oder 1,3-Propandiamin- oder Triethylentetramin-Lösung mit und ohne Alkali- oder Erdalkalizusatz zur Reaktion bringt.

Derartige Borosilikatzeolithe können ebenfalls hergestellt werden, wenn man die Reaktion statt in wässriger Aminlösung in etherischer Lösung, z.B. Diethylenglykoldimethylether, oder in alkalischer Lösung durchführt.

Die so hergestellten Alumino- und Borsilikatzeolithe werden nach ihrer Isolierung, Trocknung bei 100 bis 160°C, vorzugsweise 110°C, und Calcination bei 450 bis 550°C, vorzugsweise 500°C, mit einem Bindemittel im Verhältnis 90:10 bis 40:60 Gew.% zu Strängen oder Tabletten verformt. Als Bindemittel eignen sich diverse Aluminiumoxide, bevorzugt Boehmit, amorphe Aluminosilikate mit einem $SiO_2/Al_2O_3$-Verhältnis von 25:75 bis 95:5, bevorzugt 75:25, Siliciumdioxid, bevorzugt disperses $SiO_2$, Gemische aus hochdispersem $SiO_2$ und hochdispersem $Al_2O_3$, hochdisperses $TiO_2$ sowie Ton. Nach der Verformung werden die Extrudate oder Presslinge bei 110°C/16 h getrocknet und bei 500°C/16 h calciniert.

Eine besondere Ausführungsform besteht darin, dass der isolierte Alumino- bzw. Borosilikatzeolith direkt nach der Trocknung verformt wird und erstmals nach der Verformung einer Calcination unterworfen wird.

Weiterhin können Aluminosilikatzeolithe des Y-Typs verwendet werden, die aus Kieselsol (29% $SiO_2$) und Natriumaluminat in wässrigem Medium hergestellt wurden. Diese Aluminosilikatzeolithe können vor ihrem Einsatz ebenfalls mit Bindern verformt werden.

Nach der Desaktivierung der zeolithischen Katalysatoren durch Koksabscheidung während der oben beanspruchten Reaktion lassen sich diese durch Abbrennen der Koksablagerung mit Luft oder mit einem Luft/$N_2$-Gemisch bei 400 bis 550°C, bevorzugt 500°C, in einfacher Weise regenerieren und erhalten dadurch ihre Anfangsaktivität zurück.

Zur Erhöhung der Selektivität, der Standzeit und der Anzahl an Regenerierungen lassen sich unterschiedliche Modifizierungen an diesen zeolithischen Katalysatoren vornehmen.

Eine Modifizierung der Katalysatoren besteht darin, dass man den unverformten oder dem verformten Zeolithen mit Alkalimetallen wie Na, Erdalkalimetallen wie Ca, Mg, Erdmetallen wie B, Tl, Übergangsmetallen wie Mn, Fe, Mo, Cu, Zn und seltenen Erdmetallen wie La, Ce ionenaustauschen bzw. dotieren kann.

Eine besondere Ausführungsform besteht darin, dass man den verformten Pentasilzeolithen in einem Steigrohr vorlegt und bei 20 bis 200°C z.B. ein Halogenid oder ein Nitrat der voranbeschriebenen Metalle überleitet. Ein derartiger Ionenaustausch kann z.B. an der Wasserstoff-, Ammonium- und Alkaliform des Zeolithen vorgenommen werden.

Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wässriger oder alkoholischer Lösung imprägniert.

Sowohl an einen Ionenaustausch als auch an eine Imprägnierung schliesst sich zumindest eine Trocknung, wahlweise eine abermalige Calcination an.

Bei metalldotierten Zeolithen ist eine Nachbehandlung mit Wasserstoff möglich. Eine weitere Möglichkeit der Modifizierung besteht darin, dass man das zeolithische Material – verformt oder unverformt – einer Behandlung mit Säuren wie Salzsäure, Flusssäure und Phosphorsäure unterwirft.

Eine besondere Ausführungsform besteht darin, dass man das zeolithische Pulver vor seiner Verformung mit Flusssäure 0,001 n bis 2 n, bevorzugt 0,05 bis 0,5 n, 1 bis 3 Stunden unter Rückfluss behandelt. Nach Abfiltrieren und Auswaschen wird bei 100 bis 160°C getrocknet und bei 450 bis 600°C calciniert.

Eine weitere besondere Ausführungsform liegt in einer HCl-Behandlung der Zeolithe nach ihrer Verformung mit Bindemittel. Hierbei wird der Zeolith 1 bis 3 Stunden zwischen 60 und 80°C in einer 3- bis 25%igen, insbesondere mit einer 12- bis 20%igen Salzsäure behandelt, anschliessend ausgewaschen, bei 100 bis 160°C getrocknet und bei 450 bis 600°C calciniert. Auch kann durch partielle Verkokung (pre-coke) die Aktivität des Katalysators für ein Selektivitätsoptimum des gewünschten Reaktionsproduktes eingestellt werden.

Die hier beschriebenen Katalysatoren werden wahlweise als 2- bis 4-mm-Stränge oder als Tabletten mit 3 bis 5 mm Durchmesser oder als Pulver mit Teilchengrösse von 0,1 bis 0,5 mm eingesetzt.

Die für die Isomerisierung gewählten Reaktionsbedingungen sind in der Gasphase 180 bis 400°C, vorzugsweise 250 bis 300°C, und eine Belastung WHSV = 0,1 bis 10 $h^{-1}$ (d Diacyloxybuten/g Katalysator und Stunde).

Auch in der Flüssigphase bei 100 bis 170°C lässt sich die Isomerisierung der Diacyloxybutene durchführen.

Folgende Beispiele veranschaulichen die Erfindung.

Beispiel 1

Katalysator A

Der Aluminosilikatzeolith des Pentasil-Typs wird unter hydrothermalen Bedingungen bei autogenem Druck und 150°C aus 65 g $SiO_2$ (Aerosil 200), 20,3 g $Al_2(SO_4)_3 \times 18\ H_2O$ in 1 kg einer wässrigen 1,6-Hexandiamin-Lösung (Mischung 50:50 Gew.%) in einem Rührautoklaven synthetisiert. Nach Abfiltrieren und Auswaschen wird das kristalline Reaktionsprodukt bei 110°C/34 h getrocknet und bei 500°C/24 h calciniert. Dieser Aluminosilikatzeolith enthält 91,6 Gew.% $SiO_2$ und 4,6 Gew.% $Al_2O_3$.

Dieses Produkt wird mit Boehmit im Gewichtsverhältnis 60:40 zu 2-mm-Strängen verformt und bei 110°C/16 h getrocknet und bei 500°C/24 h calciniert.

Katalysator B

Katalysator B wird hergestellt wie Katalysator A, indem lediglich die wässrige 1,6-Hexandiamin-Lösung durch wässrige 1,3-Propandiamin-Lösung gleicher Konzentration ersetzt wird. Dieser Aluminiumsilikatzeolith setzt sich zusammen aus 90 Gew.% $SiO_2$ und 3,5 Gew.% $Al_2O_3$. Die Kristallitgrösse beträgt 10 bis 12 μ.

Katalysator C

Die Herstellungsweise des Katalysators C entspricht der des Katalysators B. Der Aluminosilikatzeolith setzt sich zusammen aus 90,6 Gew.% $SiO_2$ und 3,9 Gew.% $Al_2O_3$ und seine Kristallitgrösse beträgt 0,1 bis 0,5 μ.

Katalysator D

Der Borzeolith des Pentasil-Typs wird in einer hydrothermalen Synthese aus 64 g $SiO_2$ (Aerosil 200), 12,2 g $H_3BO_3$, 800 g einer wässrigen 1,6-Hexandiamin-Lösung (Mischung 50:50 Gew.%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borosilikatzeolith setzt sich zusammen aus 94,2 Gew.% $SiO_2$ und 2,32 Gew.% $B_2O_3$.

Mit diesem Material werden durch Verformen mit Boehmit im Gewichtsverhältnis 60:40 2-mm-Stränge hergestellt, die bei 110°C/16 h getrocknet und bei 500°C/24 h calciniert wurden.

Katalysator E

Katalysator E wird aus Katalysator A hergestellt, indem dieser mit 0,88 molarer $Cu(NO_3)_2 \times 2\ H_2O$ imprägniert wird.

Katalysator F

Katalysator F wird durch Salzsäure-Behandlung des Katalysators C (1 n Salzsäure, Rückfluss, 1 Stunde) hergestellt. Nach Auswaschen mit Wasser wird bei 100°C/16 h getrocknet und bei 500°C/5 h calciniert.

Versuche 1 bis 10

Die Versuche 1 bis 10 veranschaulichen die Verwendung der oben beschriebenen Katalysatoren für die gegenseitige Umwandlung von 1,4-Diacyloxy-2-butenen und 3,4-Diacyloxy-1-butenen in der Gasphase (Tabelle). Als leichtsiedende Nebenprodukte der Reaktion sind z.B. Essigsäure, Butadienylacetate zu nennen.

Einsatzstoff gemäss Formel I enthält 92,3 Gew.% trans-1,4-Diacetoxybuten und 5,9 Gew.% cis-1,4-Diacetoxybuten.

Einsatzstoff gemäss Formel II setzt sich zusammen aus 99,6 Gew.% 3,4-Diacetoxybuten und 0,4 Gew.% trans- und cis-1,4-Diacetoxybuten.

Versuchsdurchführung erfolgt in einem Rohrreaktor unter isothermen Bedingungen. Der Einsatzstoff wird auf einer Vorheizstrecke verdampft, bevor er mit dem Katalysator in Kontakt kommt. Die Reaktionsprodukte werden kondensiert und durch GC-Analytik identifiziert.

| Versuch | 1 | 2 | 3 | 4 | 5 | 6 | 7 | 8 | 9 | 10 |
|---|---|---|---|---|---|---|---|---|---|---|
| Katalysator | A | A | E | B | C | F | D | D | D | E |
| Einsatzstoff | 1,4-DAOB | 1,4-DAOB | 1,4-DAOB | 1,4-DAOB | 1,4-DAOB | 1,4-DAOB | 1,4-DAOB | 1,4-DAOB | 3,4-DAOB | 3,4-DAOB |
| Temperatur | 300°C | 300°C | 300°C | 300°C | 300°C | 300°C | 300°C | 300°C | 200°C | 300°C |
| WHSV | 6,2 $h^{-1}$ | 3,1 $h^{-1}$ | 6,2 $h^{-1}$ | 6,2 $h^{-1}$ | 6,2 $h^{-1}$ | 3,1 $h^{-1}$ | 6,2 $h^{-1}$ | 3,1 $h^{-1}$ | 6,2 $h^{-1}$ | 6,2 $h^{-1}$ |
| Produkte Gew.% | | | | | | | | | | |
| trans-1,4 BEDA | 53,2 | 38,3 | 55,2 | 64,2 | 60,1 | 34,6 | 29,5 | 42,2 | 18,4 | 11,3 |
| cis-1,4 BEDA | 5,3 | 5,4 | 7,7 | 5,5 | 5,0 | 5,7 | 5,8 | 6,0 | 1,55 | 1,5 |
| 3,4 BEDA | 26,3 | 37,0 | 21,7 | 16,2 | 17,0 | 36,2 | 25,5 | 26,6 | 75,3 | 82,1 |
| Nebenprodukte | 12,2 | 15,9 | 13,8 | 11,2 | 14,0 | 21,4 | 34,6 | 22,0 | 4,9 | 4,8 |

DAOB = Diacetoxybuten
BEDA = Butendioldiacetat

Beispiel 2

Dieses Beispiel befasst sich mit der Durchführung der Reaktion 1,4-Diacetoxy-2-buten zu 3,4-Diacetoxybuten in Flüssigphase in Gegenwart von Katalysator A. Ein beheiztes Quarzrohr von 4 cm Durchmesser und 40 cm Länge wird mit 184 g Katalysator A gefüllt. Stündlich werden 0,11 l 1,4-Diacetoxy-2-buten bei einer Temperatur von 228°C durch dieses Reaktionsrohr gepumpt.

Bei einer Verweilzeit von 2,5 Stunden erhält man ein Reaktionsgemisch, das 77 Gew.% 1,4-Diacetoxy-2-buten, 13 Gew.% 3,4-Diacetoxybuten und 10 Gew.% Nebenprodukte enthält.

## Patentansprüche

1. Verfahren zur wechselseitigen Umwandlung von Diacyloxybutenen in der Gasphase oder in flüssiger Phase bei Temperaturen von 100 bis 350°C unter normalem oder erhöhtem Druck in Gegenwart von Katalysatoren, dadurch gekennzeichnet, dass man die wechselseitige Umwandlung von 1,4-Diacyloxy-2-buten der Formel I

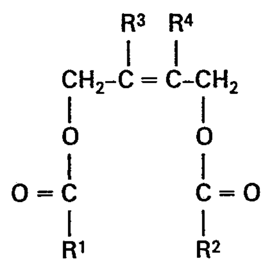

I,

und von 3,4-Diacyloxy-1-buten der Formel II

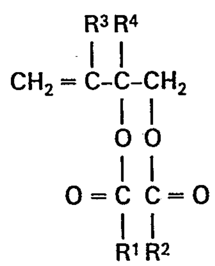

II,

in denen $R^1$ und $R^2$ gleich oder verschieden sein können und Wasserstoff, Alkylreste mit 1 bis 3 Kohlenstoffatomen, $R^3$ und $R^4$ gleich oder verschieden sein können und Wasserstoff oder Methylgruppen bedeuten, in Gegenwart von Zeolithen als Katalysatoren ausführt.

2. Verfahren nach Anspruch 1, dadurch gekennzeichnet, dass man als Katalysatoren Zeolithe des Pentasil-Typs verwendet.

3. Verfahren nach Anspruch 1 und 2, dadurch gekennzeichnet, dass man als Katalysatoren Aluminosilikatzeolithe verwendet.

4. Verfahren nach Anspruch 1 und 2, dadurch gekennzeichnet, dass man als Katalysatoren Borosilikatzeolithe verwendet.

5. Verfahren nach Anspruch 1, dadurch gekennzeichnet, dass man als Katalysatoren Aluminiumsilikatzeolithe des Y-Typs verwendet.

## Claims

1. A process for converting diacyloxybutenes into one another in the gas phase or liquid phase at from 100 to 350°C under atmospheric or superatmospheric pressure in the presence of a catalyst, wherein a 1,4-diacyloxybut-2-ene of the formula I

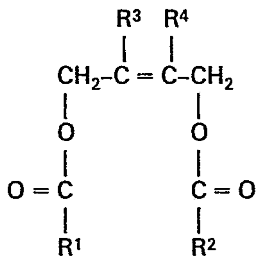

I,

and a 3,4-diacyloxybut-1-ene of the formula II

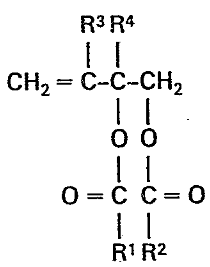

II,

where $R^1$ and $R^2$ can be identical or different and are each hydrogen or alkyl of 1 to 3 carbon atoms, and $R^3$ and $R^4$ can be identical or different and are each hydrogen or methyl, are converted into one another in the presence of a zeolite as a catalyst.

2. A process as claimed in claim 1, wherein the catalyst used is a zeolite of the pentasil type.

3. A process as claimed in claims 1 and 2, wherein the catalyst used is an aluminosilicate zeolite.

4. A process as claimed in claims 1 and 2, wherein the catalyst used is a borosilicate zeolite.

5. A process as claimed in claim 1, wherein the catalyst used is an aluminosilicate zeolite of the Y type.

## Revendications

1. Procédé de transformation réciproque de diacyloxybutènes en phase gazeuse ou en phase liquide, à des températures de 100 à 350°C, sous pression normale ou élevée et en présence de catalyseurs, caractérisé en ce qu'on effectue en présence de zéolithes servant de catalyseurs la transformation réciproque de 1,4-diacyloxy-2-butène de formule I

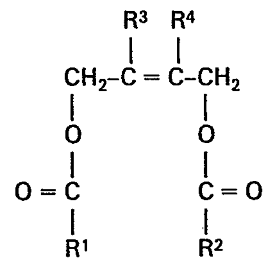

I,

et de 3,4-diacyloxy-1-butène de formule II

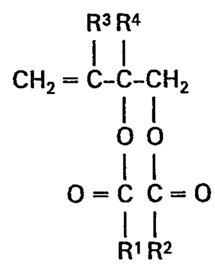

II,

dans lesquelles $R^1$ et $R^2$ peuvent être identiques ou différents et représentent des atomes d'hydrogène, des radicaux alkyle à 1–3 atomes de carbone, $R^3$ et$^4$ peuvent être identiques ou différents et représentent des atomes d'hydrogène ou des groupes méthyle.

2. Procédé selon la revendication 1, caractérisé en ce qu'on utilise comme catalyseurs des zéolithes du type pentasil.

3. Procédé selon la revendication 1 ou 2, caractérisé en ce qu'on utilise comme catalyseurs des zéolithes aluminosilicates.

4. Procédé selon la revendication 1 ou 2, caractérisé en ce qu'on utilise comme catalyseurs de zéolithes borosilicates.

5. Procédé selon la revendication 1, caractérisé en ce qu'on utilise comme catalyseurs des zéolithes aluminosilicates du type Y.